(19) Europäisches Patentamt

European Patent Office

Office européen des brevets

(11) Publication number: **0 050 459**
**B1**

(12) **EUROPEAN PATENT SPECIFICATION**

(45) Date of publication of patent specification: **20.02.85**

(51) Int. Cl.⁴: **A 61 M 5/16,** A 61 M 1/02,
A 61 M 5/14

(21) Application number: **81304729.7**

(22) Date of filing: **12.10.81**

(54) **Spike connector.**

(30) Priority: **22.10.80 US 199140**

(43) Date of publication of application:
**28.04.82 Bulletin 82/17**

(45) Publication of the grant of the patent:
**20.02.85 Bulletin 85/08**

(84) Designated Contracting States:
**BE DE FR GB SE**

(56) References cited:
**WO-A-80/02506**
**DE-A-2 221 564**
**DE-U-7 702 734**
**FR-A- 601 688**
**FR-A-2 375 870**
**US-A-2 746 455**
**US-A-2 989 053**
**US-A-3 868 965**
**US-A-4 173 223**
**US S 229 518**

(73) Proprietor: **Travenol European Research and Development Centre (Teradec)**
**Parc Industrial Rue du Progies 7**
**B-1400 Nivelles (BE)**

(72) Inventor: **Kersten, Jean**
**35c, Chaussee de Bruxelles**
**B-7931, Villers St. Amand (BE)**

(74) Representative: **MacGregor, Gordon et al**
**ERIC POTTER & CLARKSON 14 Oxford Street**
**Nottingham, NG1 5BP (GB)**

Courier Press, Leamington Spa, England.

## Description

### Technical Field

This invention concerns a spike connector that may be used in the medical field, particularly in conveying fluid from a fluid source to a patient. The spike connector of the present invention is a type having a main body portion and a hollowed spike extending therefrom for insertion into the stopper of the fluid source.

### Background Art

Known in the art are integrally molded spike connectors for coupling a drip chamber to the stopper of a fluid source. An example of one type of spike connector, commonly referred to as an airway connector, is illustrated in U.S. Design Patent No. US—S—229,518 issued December 4, 1973. Other spike connectors, primarily for use with collapsible plastic containers, do not utilize a filtered airway.

FR—A—601688 is of background interest but is not addressed to the problems which the present invention seeks to overcome. This specification discloses a spike connector for preventing gas escaping from a gas-charged beverage in a container once the container has been opened. The connector comprises a hollowed pin for piercing a stopper of the container, a valve in a main body portion, and a cap for adjusting the valve. A pair of arms protrude from the cap to facilitate relative rotation of the cap and the main body and spike.

The configuration of certain prior art spike connectors has made it difficult for an operator to have access to the stopper once the spike of the spike connector has been inserted into the stopper. For example, while certain stoppers carry an injection site, they block the injection site from the operator once the spike of the spike connector is inserted into the stopper.

Certain prior art spike connectors are configured to become relatively flush with the stopper once the spike is inserted into the stopper. This flush engagement may be deleterious because a leaking liquid may collect on the surface which engages the stopper. One prior art type of spike connector, licensed by American Hospital Supply Corporation in Italy, has a flat surface which engages the stopper with the flat surface forming the periphery of an open volume. This enables the collection of solution on the flat surface and in the open volume, and thereby increases chances of contamination.

### Disclosure of the Invention

It is an object of the present invention to provide a spike connector in which the main body portion has only an extremely small amount of contact with the stopper so as to avoid surface area which may carry contaminants.

Another object of the present invention is to provide a spike connector that enables relatively good access to an injection site on the stopper, after the spike of the spike connector is inserted into the stopper.

A further object of the invention is to provide a spike connector that has a main body portion with a very small amount of contact with the stopper, enables relatively good access to an injection site carried by the stopper, yet is relatively efficient in its use of plastic.

Another object of the present invention is to provide a spike connector that has a spike which enters easily into the stopper but maintains frictional compression with the stopper once the spike is fully inserted thereinto.

Other objects and advantages of the invention will become apparent as the description proceeds.

In accordance with the present invention, a spike connector is provided having a main body portion and a hollowed spike extending therefrom for insertion into the stopper of a fluid source and for conveyance of the fluid from the source through the spike and main body portion. The main body portion includes a pair of wings which extend upwardly in the direction of the spike but outwardly therefrom. The wings extend away from each other. The undersides of the wings are arcuately constructed being concavely shaped to enable an operator to grasp the main body portion at the undersides. The tops of the wings serve to space the body, other than the wing tops, away from the fluid source stopper when the spike is inserted into the stopper.

In the illustrative embodiment, the spike connector is molded in an integral, one-piece construction, with each wing forming an included angle of about 45° between the wing and the axis of the spike. The main axis of both wings and the spike are coplanar.

In the illustrative embodiment, the spike has a relatively polished surface adjacent its tip as compared to the surface adjacent the main body portion, in order to enable easy insertion of the spike into the stopper but good frictional engagement of the spike with the stopper once the spike is fully inserted into the stopper.

A more detailed explanation of the invention is provided in the following description and claims, and is illustrated in the accompanying drawings.

### Brief Description of Drawings

Figure 1 is a perspective view of a spike connector constructed in accordance with the principles of the present invention.

Figure 2 is a top plan view thereof.

Figure 3 is a cross-sectional elevation thereof, taken along the plane of the line 3—3 of Figure 2.

Figure 4 is a cross-sectional elevation thereof, taken along the plane of the line 4—4 of Figure 2.

Figure 5 is a front elevational view of an airway connector forming a modified embodiment of the present invention.

Figure 6 is a side elevational view thereof.

### Detailed Description of the Illustrative Embodiments

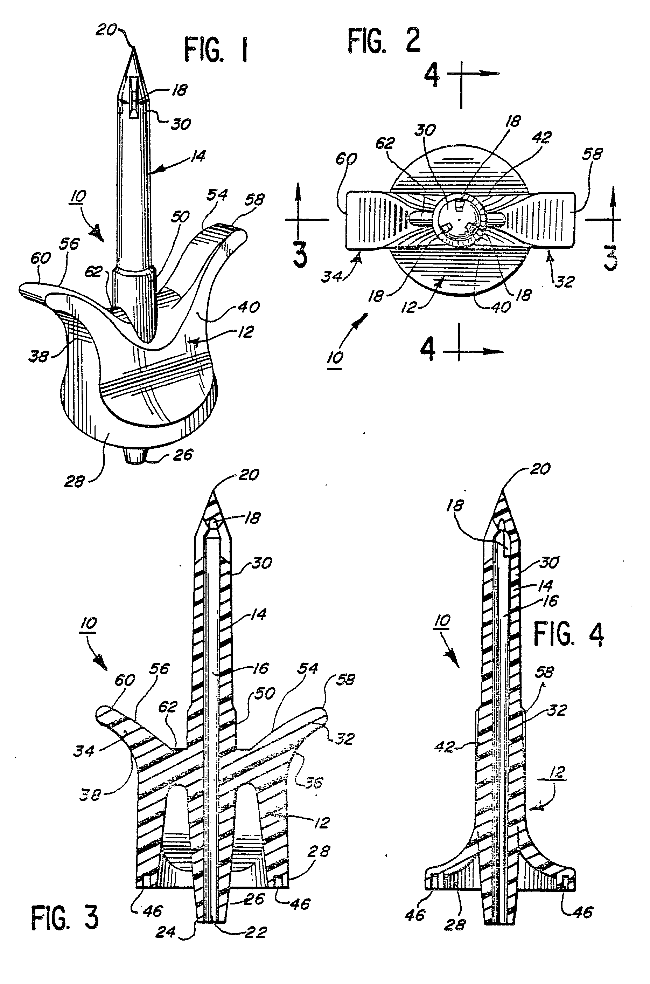

Referring to Figures 1—4, a spike connector 10 is shown therein having a main body portion 12 and a hollowed spike 14 extending from the main body portion. The hollowed spike 14 is adapted for insertion into the stopper of a fluid source, such as a parenteral liquid container in the form of a collapsible plastic container. Main body portion 12 and spike 14 define a bore 16 which extends from an opening 18 adjacent spike tip 20 to outlet opening 22 at the distal end 24 of drip tube 26, for conveying the fluid from the container through opening 18, via bore 16 and through outlet opening 22, typically to a drip chamber that is connected to the bottom 28 of main body portion 12.

Spike connector 10 is preferably molded of polypropylene in an integral, one-piece construction. When the molding is completed, a surface area 30 from tip 20 downward to between about 1.0 centimeter and 1.5 centimeter, is polished. This provides surfaces 30 with a relatively polished, smooth surface finish in contrast to the surface finish of the remainder of the spike, thereby allowing relatively easy entry of the spike into the stopper but providing good retention of the spike within the stopper once it is inserted, by reason of the frictional compression provided by the less polished lower portion.

Main body portion 12 includes a pair of wings 32, 34 each of which extends upwardly in the direction of the spike 14 but outwardly therefrom, preferably forming an included angle of about 45° between the respective wing and the spike axis. In the illustrative embodiment, the main axes of both wings 32 and 34, and the axis of spike 14 are coplanar.

Main body portion 12 has four sides 36, 38, 40 and 42. Sides 36 and 38 form the undersides of wings 32 and 34, respectively, and are arcuate, being concavely shaped, to provide an economically and functionally shaped comfortable finger guard. The sides 36 and 38 and the top portions thereof are narrow and generally planar while the bottom portions of sides 40 and 42 curve outwardly away from the spike. The bottom 38 of the main body portion 12 has a generally circular cross-sectional configuration perpendicular to the spike, and a slot 46 is provided to receive the top portion of the drip chamber. Drip tube 26 has an outlet opening 22 that provides a drop rate of about 20 drops per minute.

The lower portion 50 of spike 14 has a greater external diameter, so that the spike may be utilized with containers having a larger than normal stopper opening. In the illustrative embodiment, spike 14 is provided with three equally spaced openings 18 about the spike tip and in communication with bore 16.

Top surfaces 54 and 56 of wings 32 and 34, respectively, are smooth and curved to avoid dust and fluid collection. Wing tips 58 and 60 are spaced a relatively great distance from lower surface 62 and tips 58 and 60 will be the only contact points of the main body portion against the stopper when the spike is inserted into the stopper. This substantial lack of surface contact between the main portion and the stopper is useful in preventing the collection of leaking liquid on the main body portion. In addition, by utilizing extending wings 32 and 34, bottom 28 of the main body portion 12 is at a relatively far distance away from the stopper, without having to utilize a substantial amount of plastic, and enabling the operator to have relatively good access to an injection site carried by the stopper. By utilizing top portions of sides 40 and 42 that are relatively flat surfaces, the operator may write on such flat surface with a marking pen, if so desired, while the curved lower surface of sides 40 and 42 enable easy removal of the spike connector from the stopper.

It can be seen that wings 32 and 34 in cooperation with the lower portion of the main body portion operate to provide a finger grip that is efficiently designed for easy insertion and removal of the spike connector from the stopper. At the same time, wings 32 and 34 are operable to prevent touch contamination of the stopper. Further, the relatively narrow sides 40 and 42 plus the distance of the bottom of the main body portion from the top thereof aid in enabling the operator to have good access to an injection site carried by the stopper for supplementary medication additions or use of a separate airway. Still further, the wings have very small contact points with the stopper when a spike is inserted into the stopper, and thus contamination by reason of the liquid pooling along the top of the main body portion is alleviated.

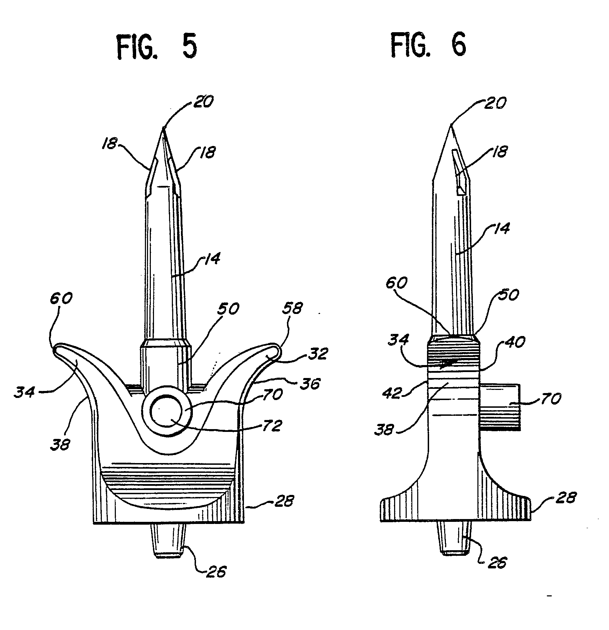

An airway connector, constructed in accordance with the principles of the present invention, is illustrated in Figures 5 and 6. Identical reference numerals are used in Figures 5 and 6 for structural components which are identical to those utilized in the Figures 1—4 embodiment. However, the airway connector of Figures 5 and 6 utilizes a spike 14 which carries a separate fluid bore and airway bore, with the airway bore communicating with a filtered airway opening 70. Although airway opening 70 is illustrated at a right angle with respect to spike 14, it is to be understood that the airway opening may extend at other than a right angle with respect to spike 14. The airway opening 17 surrounds a hydrophobic airway filter 72, preferably formed of Porex filter material. A cap or plug may be utilized to close and seal airway 70 when the airway is not used, for example, when the connector is issued with a collapsible plastic container.

All of the benefits and the results concomitant with the spike connector described in connection with Figures 1—4 are also concomitant with respect to the airway spike connector described in connection with Figures 5 and 6.

Although illustrative embodiments of the invention have been shown and described, it is to be understood that various modifications and substitutions may be made by those skilled in the art without departing from the novel spirit and scope of the present invention.

## Claims

1. A spike connector having a body (12), a hollow spike (14) extending therefrom at one side for insertion into a stopper of a fluid source, and fluid connection means (26) at the other side of the body and in communication with the hollow spike, the body being provided with an outwardly extending part (32, 34) for facilitating manual insertion of the spike in the stopper, the outwardly extending part comprising a pair of wings (32, 34) projecting outwardly to opposite sides of the body (12) and also in the direction of the spike, characterized in that, in use, the wing tops contact the stopper to space the remainder of the body away from the stopper, each of the surfaces (36, 38) of the wings, opposite to those facing the spike, being concavely shaped to facilitate manual gripping of the surfaces.

2. A spike connector according to Claim 1, wherein said main body portion has at its end distal from the spike a generally circular cross-sectional configuration perpendicular to the spike, and including means adjacent the circular cross-section portion (28) for receiving a drip chamber.

3. A spike connector according to Claim 1 or 2 which is moulded of polypropylene in a one-piece construction.

4. A spike connector according to Claim 1, 2 or 3, wherein each wing forms an included angle of about 45° between the wing and the axis of the spike.

5. A spike connector according to any preceding Claim, wherein the main axes of both wings and the spike are co-planar.

6. A spike connector according to any preceding Claim, wherein the main body portion (12) has four sides, two sides of the main body portion forming the undersides of the wings and the other two sides of the main body portion being narrow and generally planar adjacent the spike but curving outwardly away from the spike.

7. A spike connector according to any preceding Claim, wherein said spike (14) has a relatively polished surface adjacent its tip (20) as compared to the surface adjacent the main body portion (12).

8. A spike connector according to Claim 7, wherein said relatively polished surface extends from 1.0 centimetre to 1.5 centimetres from the tip (20).

## Revendications

1. Connecteur à aiguille comportant un corps (12), une aiguille creuse (14) qui s'étend à partir d'une face dudit corps pour être introduite dans le bouchon d'une source de fluide, et des moyens de liaison fluidique (26) de l'autre côté du corps et communiquant avec l'aiguille creuse, ledit corps présentant une partie qui s'étend vers l'extérieur (32, 34) pour faciliter l'introduction manuelle de l'aiguille dans le bouchon, la partie qui s'étend vers l'extérieur comprenant deux ailettes (32, 34) qui dépassent vers l'extérieur sur les côtés du corps (12) et également en direction de l'aiguille, caractérisé en ce que, à l'usage, les dessus des ailettes viennent en contact avec le bouchon pour espacer le reste du corps du bouchon, chacune des surfaces (36, 38) des ailettes opposées à celles faisant face à l'aiguille, présentant une forme concave pour faciliter la prise manuelle de ces surfaces.

2. Connecteur à aiguille suivant la revendication 1, caractérisé en ce que le corps principal présente à son extrémité opposée à l'aiguille une configuration de section sensiblement circulaire perpendiculaire à l'aiguille, et comprenant des moyens adjacents à la partie de section circulaire (28) pour recevoir une chambre de goutte à goutte.

3. Connecteur à aiguille suivant la revendication 1 ou la revendication 2, caractérisé en ce qu'il est moulé d'un seul tenant en polypropylène.

4. Connecteur à aiguille suivant les revendications 1, 2 ou 3, caractérisé en ce que chaque ailette forme avec l'axe de l'aiguille un angle inclus d'environ 45°.

5. Connecteur à aiguille suivant l'une des revendications précédentes, caractérisé en ce que les axes principaux des deux ailettes et de l'aiguille reposent dans un même plan.

6. Connecteur à aiguille suivant l'une quelconque des revendications précédentes, caractérisé en ce que le corps principal (12) présente quatre côtés, dont deux forment le dessous des ailettes et les deux autres sont étroits et sensiblement plats au voisinage de l'aiguille mais s'incurvent vers l'extérieur à distance de l'aiguille.

7. Connecteur à aiguille suivant l'une quelconque des revendications précédentes, caractérisé en ce que l'aiguille (14) a une surface relativement polie près de sa pointe (20) par rapport à sa surface voisine du corps principal (12).

8. Connecteur à aiguille suivant la revendication 7, caractérisé en ce que la surface relativement polie précitée s'étend sur une distance de 1,0 à 1,5 centimètre à partir de la pointe (20) de l'aiguille.

## Patentansprüche

1. Nadelanschluß mit einem Grundkörper (12), einer Hohlnadel (14), die an einer Seite von diesem absteht, zum Einführen in einen Verschluß eines Flüssigkeitsbehälters und mit Flüssigkeits-Verbindungs-Mitteln (26) an der anderen Seite des Grundkörpers sowie in Verbindung mit der Hohlnadel, wobei der Grundkörper ein nach außen ragendes Teil (32, 34) aufweist, zur Erleichterung des manuellen Einführens der Nadel in den Verschluß, wobei das nach außen regende Teil ein paar von Ansätzen (32, 34) aufweist, die an gegenüberliegenden Seiten des Grundkörpers (12) nach außen ragen und auch in Richtung auf die Nadel dadurch gekennzeichnet, daß bei der Benutzung die Spitzen der Ansätze den Verschluß berühren und den Rest des Grundkörpers von dem Verschluß im Abstand halten, wobei diejenigen Flächen (36, 38) der Ansätze, die den zur Nadel weisenden Flächen gegenüberliegen, konkav geformt sind, um ein manuelles Ergreifen der Flächen zu erleichtern.

2. Nadelanschluß nach Anspruch 1, bei dem der Hauptkörperabschnitt an seinem gegenüber der Spitze distalen Ende einen im wesentlichen kreisförmigen Querschnitt rechtwinklig zur Nadel aufweist und benachbart zu dem kreisförmigen Querschnittsteil (28) Einrichtungen zur Aufnahme einer Tropfkammer aufweist.

3. Nadelanschluß nach Anspruch 1 oder 2, dadurch gekennzeichnet, daß er einstückig aus Polypropylen geformt ist.

4. Nadelanschluß nach Anspruch 1, 2 oder 3, dadurch gekennzeichnet, daß jeder Ansatz zwischen sich und der Nadelachse einen Winkel von ca. 45° einschließt.

5. Nadelanschluß nach einem der vorhergehenden Ansprüche, dadurch gekennzeichnet, daß die Hauptachsen der Ansätze und der Nadel in einer Ebene liegen.

6. Nadelanschluß nach einem der vorhergehenden Ansprüche, dadurch gekennzeichnet, daß der Grundkörperabschnitt (12) vier Seiten aufweist, wobei zwei Seiten des Grundkörperabschnittes die Unterseiten der Ansätze bilden und wobei die anderen Seiten des Grundkörperabschnittes schmal sind und angrenzend zur Nadel eben, jedoch von der Nadel nach außen gekrümmt sind.

7. Nadelanschluß nach einem der vorhergehenden Ansprüche, dadurch gekennzeichnet, daß die Nadel (14) angrenzend zu ihrer Spitze (20) eine verhältnismäßig polierte Oberfläche hat, verglichen mit der an den Grundkörperabschnitt (12) angrenzenden Oberfläche.

8. Nadelanschluß nach Anspruch 7, dadurch gekennzeichnet, daß die verhältnismäßig polierte Oberfläche sich von der Spitze (20) von 1,0 cm bis 1,5 cm erstreckt.

FIG. 1

FIG. 2

FIG. 3

FIG. 4

# FIG. 5

# FIG. 6